# EUROPEAN PATENT SPECIFICATION

(11) **EP 4 268 754 B1**
(45) Date of publication and mention of the grant of the patent: **29.05.2024**
(21) Application number: 22170453.9
(22) Date of filing: 28.04.2022
(51) Int. Cl.: A61B 18/22, G02B 6/00

(54) **WAVEGUIDE FOR TREATING INNER WALLS OF INNER PATHWAYS OF A HUMAN BODY**
WELLENLEITER FÜR DIE BEHANDLUNG DER INNENWÄNDE DER INNENBAHNEN EINES MENSCHLICHEN KÖRPERS
GUIDE D'ONDES POUR TRAITER DES PAROIS INTERNES DE VOIES INTERNES D'UN CORPS HUMAIN

(43) Date of publication of application: 01.11.2023
(73) Proprietor: Oberon GmbH, 15745 Wildau (DE)
(72) Inventor: Schrobback, Mark, 15713 Königs Wusterhausen (DE)
(74) Representative: ETL IP Patent- und Rechtsanwaltsgesellschaft mbH

(56) References cited:
- EP-A1- 0 292 621
- WO-A1-91/02561
- WO-A1-2011/096629
- WO-A1-2017/142232
- CN-A- 113 017 825
- US-A- 6 152 951
- US-A1- 2003 118 302
- US-A1- 2005 165 462
- US-A1- 2006 104 593
- US-A1- 2008 177 257
- US-A1- 2008 267 561
- US-A1- 2019 008 589
- US-B1- 6 270 492

## Description

### Field of the Invention

The invention generally relates to a waveguide for treating inner walls of inner pathways of a human body with energy waves, wherein the waveguide comprises a first longitudinal end for receiving energy waves, a second longitudinal end for redirecting the energy waves that travelled from the first longitudinal end through the waveguide to the second longitudinal end, the first and second longitudinal ends being opposite ends of the waveguide along a longitudinal extend of the waveguide, and a lateral side that extends from the first longitudinal end to the second longitudinal end along the longitudinal extend. The second longitudinal end comprises at least one redirection surface provided by the waveguide. The redirection surface is unpolished.

### Technological Background

Waveguides, such as glass fibers, are used to transport energy waves, such a laser or other light, into the human body in order to treat inner walls of inner pathways of a human body. The inner walls may by the inner lining of blood vessels or of the trachea or of the bronchus or of the esophagus etc. The energy waves heat up the inner walls to thermally treat the inner walls.

Further, ends of known waveguide direct the energy waves onto the inner walls. To do so, the known waveguides have reflecting surfaces, which reflect the energy waves to narrow areas of the inner walls. To do so, total reflection is used at the ends.

Waveguides for treating inner walls of inner pathways of a human body with energy waves are disclosed in US 2008/267561 A1, US 2006/104593 A1, CN 113 017 825 A, WO 91/02561 A1, WO 2011/096629 A1, US6 152 951 A, DE 0 292 621 A1, US 6 270 492 B1, US 2019/008589 A1, US 2008/177257 A1, US 2003/118302 A1, WO 2017/142232 A1 and US 2005/165462 A1.

However, the ends are complicated to produce. In order to provide the end with a reflective surface, the roughness of the reflective surface needs to be much lower than the wavelength of the light used. As the waveguides need to have a very small diameter to be usable also for blood vessels, the reflective surface at the end of the waveguide is not easy to handle. Further, the concentration of the energy waves onto the narrow areas may result in that the user of the waveguide has to apply the waveguide and particularly the free end thereof extremely precisely, in particular in case areas that are larger than the narrow area are to be treated.

### Description of the Invention

In view of the above-mentioned disadvantages, an object of the invention may be to provide a guide for treating inner walls of inner pathways of a human body with energy waves, that is easier to produce and to apply.

This object is achieved according to the invention defined in the appended claims in that the redirection surface comprises a predefined roughness with a roughness parameter Rₐ between 0,5 µm and 10 µm.

Due to unpolished nature of the redirection surface, the waveguide can be produced easier, as the rather small redirection surface needs not to be polished. Further, due to the unpolished nature of the redirection surface, the redirected light is directed onto a less narrow surface, which allows for an easily achievable even distribution of the energy onto the inner walls. The solution according to the invention can be further improved by the embodiments mentioned in the following, which, unless explicitly mentioned to the contrary, can be combined as desired. Each of the embodiments are advantageous on the own and may even improve waveguides with polished redirection surfaces. The embodiments and their possible advantages are elaborated below:
According to the invention, the redirection surface comprises a predefined roughness. The roughness may be predefined by machining the waveguide to form the redirection surface.

The roughness has a roughness parameter Rₐ that is between a lower value of 0,5 µm and an upper value of 10 µm, wherein the lower value may be 0,7 µm, 1 µm, 1,3 µm, 1,5 µm, 1,7 µm, 2 µm, 3 µm, 5 µm, 7 µm or less than10 µm. The upper value may be 7 µm, 5 µm, 3 µm, 2 µm, 1,7 µm, 1,5 µm, 1,3 µm, 1 µm, 0,7 µm or more than 0,5 µm. For example, the roughness parameter Rₐ may be 1,3 µm.

The amplitude of the roughness may be similar to or even greater than the wavelength of light used for treating. The light may be infrared light or visible light. For example, the wavelength may be between a lower and an upper value, wherein the lower value may be 380 nm, 400 nm, 450 nm, 495 nm, 500 nm, 550 nm, 570 nm, 590 nm, 620 nm, 750 nm, 800 nm, 1,4 µm, 2,2 µm, 3 µm, 8 µm, 15 µm or less than 1 mm.

The upper value may be 1 mm, 15 µm, 8 µm, 3 µm, 2,2 µm, 1,4 µm, 800 nm, 750 nm, 620 nm, 590 nm, 570 nm, 550 nm, 500 nm, 495 nm, 450 nm, 400 nm or more than 380 nm.

The amplitude of the roughness may correspond to the wavelengths of light used for treating multiplied with a factor of between a lower and an upper value, wherein the lower value may be greater than 1, 1,5, 2, 3, 4 or 5, and wherein the upper value may be 20, 15, 10, 7 or 6.

An advantage of this embodiment may be that light redirected by the redirection surface is redirected in a diffuse manner, which may not be limited to total reflection, but can also be used as a diffusor surface that transmits the light in a diffuse manner. Diffusing the light may result in a wider distribution of the light on the inner surface area to be treated. The redirected light may have less hard boundaries, which may allow for smoothly treating the inner surface in an easy manner.

According to an embodiment, the redirection surface is formed by grinding the second end of the waveguide and/or by laser treatment of the material of the second end of the waveguide at least on the surface. Grinding may leave characteristic machining traces like rough surfaces, such that not only the general form of the redirection surface, but also its optical properties can be produced in one processing step. Laser treatment may be performed as laser melting or vaporizing or sublimation or ablation, which allows for more complicated geometries of the redirection surface. Melting may result in a redirection surface that is capable of reflecting light in a similar manner as polished surfaces, e.g. by total reflection. Yet, microscopic differences between polished and melted and then solidifying may exist. Vaporization or sublimation or ablation may result in a redirection surface that has a or the above predefined roughness. Yet, again, only one process step is required. Alternatively or additionally, laser treatment may be used to remove material to form the redirection surface, e.g. by ablation. This may be used to create more complicated general forms of the redirection surface and, additionally, to form the redirection surface with the desired optical properties, e.g. the diffuse properties.

According to an embodiment, the redirection surface juts the lateral side in the longitudinal extend. Hence, the redirection surface may form the most extreme end of the waveguide.

An advantage of this embodiment may be that the redirection surface can be reached easily for processing the waveguide.

According to an embodiment, the redirection surface forms a recess that extends along the longitudinal extend from the second longitudinal end towards the first longitudinal end.

An advantage of this embodiment may be that sharp, acute or pointy parts of the redirection surface cannot contact the inner wall, thereby reducing the risk of affecting the inner wall in an unintended manner with low contractional effort.

According to an embodiment, the redirection surface outlines a cone shape, a pyramid shape or a wedge shape. The cone shape may a complete cone or a truncated cone. The pyramid shape may include pyramids with a polygonal base, the polygon having three, four, five or more corners and may be complete or truncated. The wedge shape may correspond to a symmetric or to an asymmetric wedge and may be complete or truncated.

An advantage of this embodiment may be that different geometries that can be adapted to the surface area to be treated, can be provided.

According to an embodiment, the second longitudinal end comprises an end face. The end face may differ from the redirection face.

An advantage of this embodiment may be that the end face may provide the possibility for further usage of the second longitudinal end, which may facilitate construction and/or applicability of the waveguide. For example, the end face may form a stop for assembling or an indicator for positioning markings on the waveguide. The markings may be formed as laser markings and aide the usage of the waveguide.

According to an embodiment, the end face is essentially aligned perpendicular to the longitudinal extend at the second longitudinal end.

An advantage of this embodiment may be that the end face can be formed by not machining a part of the second longitudinal end, i.e. a section of the core, the end of the cladding or the end of the coating, such that only the part of the second longitudinal end that is to become the redirection surface needs to be machined by e.g. grinding or laser treatment.

According to an embodiment, the end face contacts the redirection surface. An advantage of this embodiment may be that the available space at the second longitudinal end of the thin waveguide can be used efficiently.

According to an embodiment, the end face at least partly or even completely extends around the redirection surface perpendicular to the longitudinal extend of the waveguide at the second longitudinal end.

An advantage of this embodiment may be that the end face can provide an indicator for mounting other elements or for applying markings to the waveguide, e.g. by laser marking.

According to an embodiment, the end face contacts the and at least partly or even completely extends around the redirection surface perpendicular to the longitudinal extend of the waveguide at the second longitudinal end.

An advantage of this embodiment may be that the end face can define an edge of the redirection surface, which may improve producibility and/or applicability of the redirection surface. Additionally or alternatively, the end face can provide an indicator for mounting other elements or for applying markings to the waveguide, e.g. by laser marking.

According to an embodiment, the end face is arranged at a distance to the redirection surface.

An advantage of this embodiments may be that the second longitudinal end is available for forming the redirection surface over the whole diameter of the waveguide. Additionally or alternatively, the end face can provide an indicator for mounting other elements or for applying markings to the waveguide, e.g. by laser marking.

For example, the waveguide may comprise a cladded silica optical fiber, which comprises a core and a cladding around the core. The end face can be provided by the cladding of the optical fiber. The cladding can have an optical property like the refractive index that differs from the respective optical property of the core. The cladding can contact and/or cover a circumferential surface of the core at least partly. The cladding can end at and thus contact the redirection surface or can end at a distance to the redirection surface. For example, in case the redirection surface forms a recess, the cladding can end at a distance to the free end of the waveguide such that light redirected by the redirection surface is not transmitted through the cladding. If the redirection surface juts the lateral side, the cladding can end at and thus contact the redirection surface or can end at a distance to the redirection surface.

The waveguide may comprise an overclad, which may be a so-called hard-clad or a so-called polymer-clad. The cladding may be arranged between the core and the overclad. The end face can be provided by the overclad. The overclad can contact and/or cover a circumferential surface of the cladding at least partly. The overclad can end at the redirection surface or can end at a distance to the redirection surface. For example, in case the redirection surface forms a recess, the overclad can end at a distance to the free end of the waveguide such that light redirected by the redirection surface is transmitted past or by the overclad.

The end face can be formed by the core at its free end at the second longitudinal end.

The waveguide may comprise a coating. The coating, which may also be designated as jacket or buffer. The coating may contact the core. Alternatively, the cladding and/or the overclad can be arranged between the core and the coating. The coating may comprise or be a polymer, a copolymer, or a fluorine-based polymer. For example, the coating is or comprises ethylene tetrafluoroethylene or poly(ethene-co-tetrafluoroethene) and may be designated as Tefzel.

The coating may be arranged to end at a distance from or at the redirection surface. The distance between the coating and the redirection surface may be greater than the distance between the end of the cladding and/or the overclad and the redirection surface.

The end face can be provided by the coating and in particular by an end of the coating that faces towards the second longitudinal end.

The waveguide can comprise one or more than one of the above-mentioned end faces.

According to an embodiment, an ending element is provided at the second longitudinal end. The ending element may have the redirection surface received therein. The ending element may comprise a free volume, in which the redirection surface is received.

An advantage of this embodiment may be that the ending element can protect the redirection surface from mechanical contacts, e.g. when handling the waveguide, or from body fluids. Additionally, the inner volume may provide for a medium like a liquid, s gas or a vacuum remaining in the inner volume that may improve redirecting the wave energy at the redirecting surface that contacts the medium.

According to an embodiment, the ending element comprises an ending cap, a sphere, a lens, a plate, an element formed by casting and/or a coating.

An advantage of these embodiments may be that a cap can easily receive the second longitudinal end, a sphere is a stable geometry and facilitates insertion of the second longitudinal end into the inner pathways, a lens can further shape the light redirected or transmitted by the redirection surface, a plate is easy to produce and to handle, elements formed by casting are easily produced with essential identical forms and diameters. Particularly, the element formed by casting can be formed by co-casting, in which the element is broad into contact with the waveguide during the casting process. Coatings require very little space. For example, the coating can be a reflective coating, e.g. a multilayer coating that may comprise dielectric layers or a single layer coating that may comprise or is gold.

According to an embodiment, the ending element overlaps the lateral side and/or is mounted to the end face.

An advantage of this embodiment may be that the overlapped parts of the lateral side and the ending element can be attached to each other, to provide for a stable mechanical connection and seal the second longitudinal end e.g. from body liquids. The ending element mounted to the end face may require less space and may particularly not add to the diameter of the waveguide, while optionally providing the seal when also partly overlapping the lateral side.

According to an embodiment, the lateral side that is free from overlap from the ending element and/or is mounted to the end face.

An advantage of this embodiment may be that the ending element mounted to the end face may require less space and may particularly not add to the diameter of the waveguide.

According to an embodiment, a maximum diameter of the ending element and a diameter of the waveguide are essentially identical.

An advantage of this embodiment may be that the second longitudinal end can easily be introduced into the inner pathway.

### Brief Description of the Drawings

The invention is described hereinafter in more detail and in an exemplary manner using advantageous embodiments and with reference to the drawings. The described embodiments are only possible configurations in which, however, the individual features as described above can be provided independent of one another or can be omitted in the drawings:
For elements of an exemplary embodiment, which correspond in form and/or function to elements of another exemplary embodiment, the same reference numerals are used.
- Figure 1: a first exemplary embodiment of the waveguide,
- Figure 2: another exemplary embodiment of the waveguide,
- Figure 3: yet another exemplary embodiment of the waveguide,
- Figure 4: yet another exemplary embodiment of the waveguide,
- Figure 5: yet another exemplary embodiment of the waveguide,
- Figure 6: yet another exemplary embodiment of the waveguide,
- Figure 7: yet another exemplary embodiment of the waveguide,
- Figure 8: yet another exemplary embodiment of the waveguide.

### Description of Exemplary Embodiments

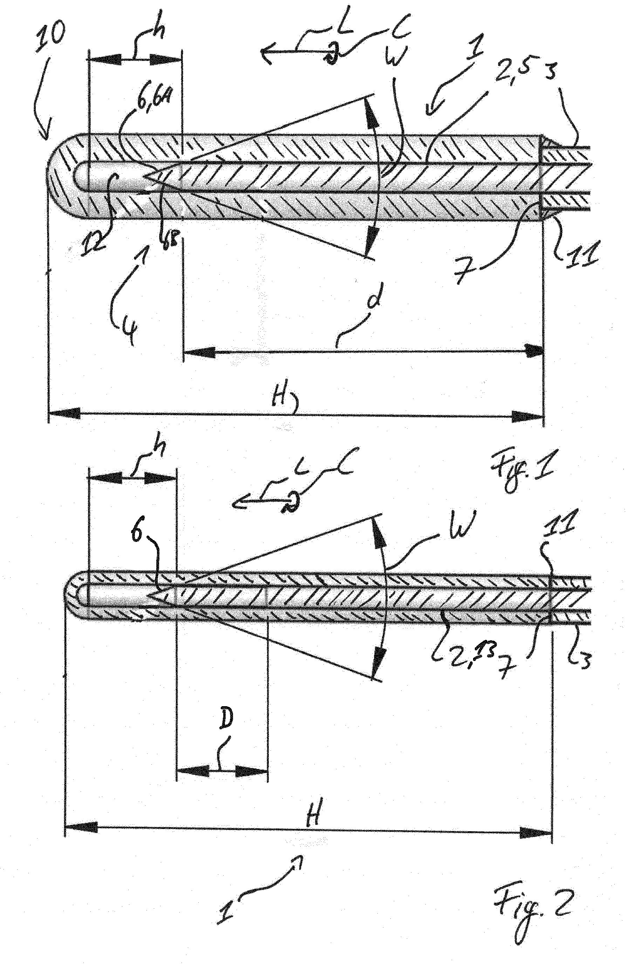

Figure 1 shows a first exemplary embodiment of a waveguide 1 for treating inner walls of inner pathways of a human body with energy waves in a schematic cross-sectional view. The waveguide 1 may comprise a core 2 and a coating 3, through which the core 2 extends. The core 2 may be formed by a polymer or by silica glass. The core 2 may comprise or be provided with a cladding that may also be designated as an outer layer on the core 2. The cladding may comprise or by a polymer or a glass. The core 2 and the cladding may have different refractive indexes.

The waveguide 1 comprises a first longitudinal end for receiving energy waves. The first longitudinal end may be adapted to be connected to a light source, e.g. a laser. Further, the waveguide 1 comprises a second longitudinal end 4 for redirecting the energy waves that travelled from the first longitudinal end through the waveguide to the second longitudinal end 4. The first and second 4 longitudinal ends are opposite ends of the waveguide 1 along a longitudinal extend L of the waveguide 1. In case the waveguide 1 is arranged to be straight, the longitudinal extend L would be the longitudinal direction of the waveguide. Yet, as the waveguide 1 can be flexible and can be bent, for example with a bending radius of less than 200 mm, less than 150 mm, less than 100 mm, less than 75 mm, down to approximately 50 mm or even less, and as the waveguide 1 needs not to be essentially straight for the definition of the invention, the expression longitudinal extend L is used instead of the expression longitudinal direction. Moreover, the waveguide 1 and optionally the core 2 thereof comprises a lateral side 5 that extends from the first longitudinal end to the second longitudinal end 4 along the longitudinal extend L. The lateral side 5 may at least sectionwise be provided by the cladding.

The second longitudinal end 4 comprises at least one redirection surface 6 provided by the waveguide and optionally by the core 2 thereof, the redirection surface 6 being unpolished. The redirection surface 6 may be free from any cladding.

The redirection surface 6 may be formed by grinding the second end of the waveguide. The redirection surface 6 may be formed by laser treatment of the material of the second longitudinal end 4 of the waveguide 1 at least on the surface. The redirection surface 6 may comprise a predefined roughness.

The roughness may be predefined by machining the waveguide 1 to form the redirection surface 6.

The roughness has a roughness parameter Rₐ that is between a lower value of 0,5 µm and an upper value of 10 µm, wherein the lower value may be 0,7 µm, 1 µm, 1,3 µm, 1,5 µm, 1,7 µm, 2 µm, 3 µm, 5 µm, 7 µm or less than10 µm. The upper value may be 7 µm, 5 µm, 3 µm, 2 µm, 1,7 µm, 1,5 µm, 1,3 µm, 1 µm, 0,7 µm or more than 0,5 µm. For example, the roughness parameter Rₐ may be 1,3 µm.

The amplitude of the roughness may be similar to or even greater than the wavelength of light used for treating. The light may be infrared light or visible light. For example, the wavelength may be between a lower and an upper value, wherein the lower value may be 380 nm, 400 nm, 450 nm, 495 nm, 500 nm, 550 nm, 570 nm, 590 nm, 620 nm, 750 nm, 800 nm, 1,4 µm, 2,2 µm, 3 µm, 8 µm, 15 µm or less than 1 mm.

The upper value may be 1 mm, 15 µm, 8 µm, 3 µm, 2,2 µm, 1,4 µm, 800 nm, 750 nm, 620 nm, 590 nm, 570 nm, 550 nm, 500 nm, 495 nm, 450 nm, 400 nm or more than 380 nm.

The amplitude of the roughness may correspond to the wavelengths of light used for treating multiplied with a factor of between a lower and an upper value, wherein the lower value may be greater than 1, 1,5, 2, 3, 4 or 5, and wherein the upper value may be 20, 15, 10, 7 or 6.

As shown in the exemplary embodiment of figure 1, the redirection surface 6 juts the lateral side 5 in the longitudinal extend L. In other words, the redirection surface 6 protrudes from the lateral side 5 in the longitudinal extend L and/or forms an extreme longitudinal end of the waveguide 1 or of the core 5 thereof.

The redirection surface 6 may outline a cone shape, a pyramid shape or a wedge shape. Outlining one of the shapes may mean that the redirection surface 5 is the surface of the second longitudinal end 4 of the waveguide 1 or of the core 2 thereof, that, as such, has the respective shape.

In case the redirection surface 6 comprises opposite redirection surface sections 6A, 6B, an angle W may be present between the opposite redirection surface sections 6A, 6B. The opposite redirection sections 6A, 6B may be symmetrical with respect to the longitudinal extend L at the second longitudinal end 4. The angle W may open against the longitudinal extend L, i.e. away from the second longitudinal end 4.

The second longitudinal end 4 may comprise an end face 7 that is essentially aligned perpendicular to the longitudinal extend L at the second longitudinal end 4. In figure 1, the end face 7 is exemplarily shown as an end face 7 provided by the coating 3.

The end face 7 may at least partly or even completely extend around the redirection surface 6 perpendicular to the longitudinal extend L of the waveguide 1 at the second longitudinal end. For example, the end face 7 may extend in a circumferential direction C, the circumferential direction C extending perpendicular to and around the longitudinal extend L.

As depicted in the exemplary embodiment of figure 1, the end face 7 may be arranged with a distance d between the end face 7 and the redirection surface 6. The distance d may be less than or about 5 mm, up to or about 5 mm, up to or about 7 mm, up to or about 10 mm, up to or about 15 mm or even up to or about 20 mm or even more.

An ending element 10 may be provided at the second longitudinal end 4, the ending element 10 having the redirection surface 6 received therein. The ending element 10 may overlap the lateral side 5 and/or may be mounted to the end face 7. According to the exemplary embodiment of figure 1, the ending element 10 may mounted to the end face 7 and, this contacts the end face 7, by a filler material 11 that is formed and arranged to provide a smooth transition between the ending element 10 and a section of the waveguide 1 that extends away from the second longitudinal end 4. For example, the filler material 11 forms a smooth transition from the ending element 10 to the coating 3. The filler material 11 may be an adhesive such that it adheres or sticks by itself to the ending element 10 and the section of the waveguide 1 that extends away from the second longitudinal end 4.

Between the lateral side 5, which may be an outer lateral side of the core 2 or of the cladding, and the ending element 10, an adhesive may be arranged that affixes the position of ending element 10 at the second longitudinal end 4.

The lateral side of the coating 3 may be free from overlap with the ending element 10.

The outer diameter of the ending element 10 may be larger than the outer diameter of the waveguide 1, of the core 2 thereof, or of the coating 3 thereof.

A total length H of the ending element may be less than or about 20 mm, less than or about 15 mm, less than or about 10 mm, less than or about 7 mm or even less than or about 5 mm. The length H may extend along the longitudinal extend L at the second longitudinal end 4.

The ending element 10 may comprise or may be an ending cap. The ending element 10 shown in the exemplary embodiment of figure 1 is formed as an ending cap with a round and e.g. half spherical closed end and with a tubular or sleeve shaped section, that has a closed end closed by the ending cap and an open end, through which the waveguide 1 and for example the core 2 thereof extend.

The ending element 10 may comprise a free volume 12, into which the waveguide 1 and at least the core 2 thereof can be introduced, preferably essentially without play perpendicular to the longitudinal extend L. At the closed end of the ending element 10, a section of the free volume 12 may remain, which may be filled with a fluid, e.g. a liquid like water or a gas like air, or a vacuum may be present. A length h of the remaining section of the free volume 12 may extend between a contact part, at which the waveguide 1 and optionally the core 2 thereof contacts the ending element 10, and the closed end of the ending element 10 that delimits the free volume 12 in the longitudinal extend L. Yet, in case the ending element 10 comprises a straight tube-shaped part closed by a curved closed end, the length h may be between a contact part, at which the waveguide 1 and optionally the core 2 thereof contacts the ending element 10, and at end part, at which the tube-shaped part contacts the curved closed end. The redirection surface 6 may end within the tube-shaped part.

The lengths h may be less than or about 5 mm, less than or about 4 mm, less than or about 3 mm, less than or about 2 mm, less than or about 1,5 mm, less than or about 1 mm or even less.

Figure 2 shows another exemplary embodiment of the waveguide 1 in a schematic cross-sectional view. For elements, which in form and/or structure correspond to the elements of the exemplary embodiment of figure 1, the same reference numerals are used. For the sake of brevity, only the differences with respect to the exemplary embodiment of figure 1 are mentioned below.

The maximum diameter of the ending element 10 and an outer diameter of the waveguide 1 and particularly of the core 2 or of the coating 3 thereof may be essentially identical. Alternatively, the maximum diameter of the ending element 10 may be greater than the outer diameter of the waveguide 1 and particularly of the core 2 or of the coating 3 thereof may be essentially identical, as shown in figure 1.

The ending element 10 may overlap the lateral side 5 and/or may contact the end face 7 of the coating 3. Particularly, as shown in the exemplary embodiment of figure 2, the ending element 10 may smoothly transition over the end face 7 onto an outer lateral surface of the coating 3, wherein the filler material 11 optionally provides for the smooth transition. Particularly, in case the diameter of the ending element 10 essentially corresponds to the diameter of the coating 3, the filler material 11 may fill a gap, groove or notch between the ending element 10 and the coating 3, the gap, groove or notch extending in the longitudinal extend L and the circumferential direction c.

As depicted in the exemplary embodiment of figure 2, the waveguide 1 is formed with a cladding 13 on the outer lateral surface of the core 2. The cladding 13 may have a longitudinal end at the second longitudinal end 4. The longitudinal cladding end may be arranged at a distance D from the redirection surface 6. The distance D may be up to 1 mm, up to 2 mm, up to 3 mm or even up to 4 mm. Hence, the outer lateral side 5 may be the side of the core 2 or of the cladding 13. The waveguides 1 of all exemplary embodiments may be formed with or without the cladding 13. Alternatively, the cladding 13 may end at the redirection surface 6.

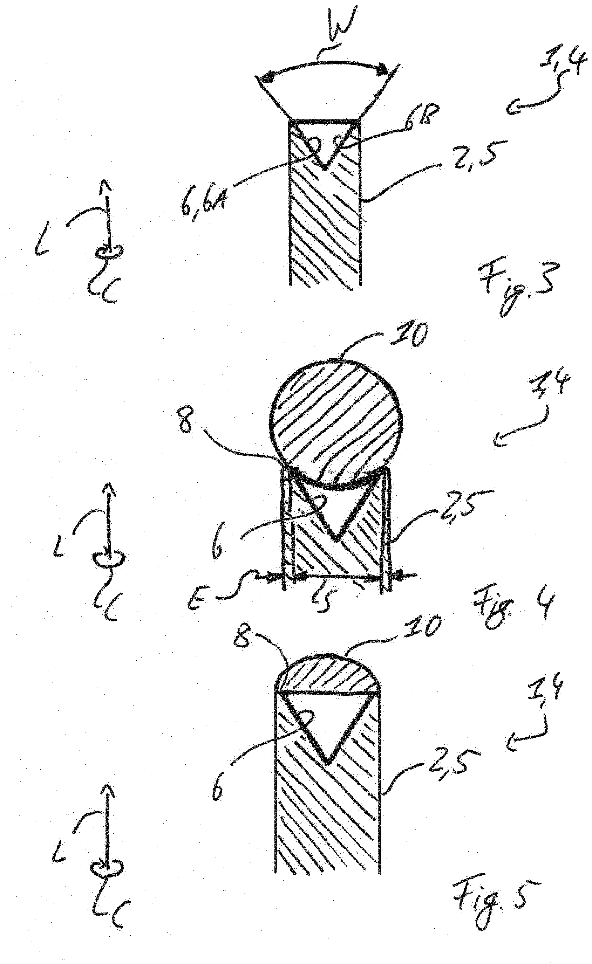

Figures 3 to 7 disclose various exemplary embodiments of the waveguide 1, wherein the core 2 is depicted while the coating 3 is omitted. For elements, which in form and/or structure correspond to the elements of the exemplary embodiments of the previous figures, the same reference numerals are used. For the sake of brevity, only the differences with respect to the exemplary embodiments of the previous figures are mentioned below.

As exemplarily shown in the exemplary embodiments of figures 3 to 7, the redirection surface 6 may form a recess that extends along or against the longitudinal extend L away from the second longitudinal end 4 and/or towards the first longitudinal end. Hence, the redirection surface 6 may be arranged between opposite sections of the lateral side 5 of the core 2.

The redirection surface 6 may outline a cone shape, a pyramid shape or a wedge shape. As one example, the figures show the cone shape.

Outlining one of the shapes may mean that the redirection surface 6 is the surface of the second longitudinal end 4 of the waveguide 1 or of the core 2 thereof, that, as such, delimits at least one side of a free volume that has the respective shape. In the latter case, the free volume may widen in the longitudinal extend L, i.e. away from the first longitudinal end and/or towards the second longitudinal end 4. In case the shape is cone shaped, the redirection surface 6 may form or delimit the lateral surface of the cone shaped free volume. The cone may be a truncated cone. In case the shape is a pyramid, the redirection surface 6 may form the lateral sides of the pyramid that form the apex of the pyramid or extend towards of the virtual shape of a truncated pyramid. In case the shape is wedge shaped, the redirection surface 6 may form the lateral sides of the pyramid that form the apex of the wedge or extend towards of the virtual ape of a truncated wedge.

Hence, as the shapes are concave, the shapes may also be designated as concave or inverse shapes.

In case the redirection surface 6 comprises opposite redirection surface sections 6A, 6B, an angle W may be present between the opposite redirection surface sections 6A, 6B. The opposite redirection sections 6A, 6B may be symmetrical with respect to the longitudinal extend L at the second longitudinal end 4. The angle W may open in the longitudinal extend L, i.e. towards the second longitudinal end 4.

Figure 3 exemplarily shows the waveguide 1 without an ending element 10. Hence, the redirection surface 6 opens in the longitudinal extend L at the second longitudinal end 4.

Figure 4 shows the waveguide 1 with an optional ending element 10. The lateral side 5 of the core 2 may be free from overlap from or free from contact with the ending element 10.

Figure 4 exemplarily shows the waveguide 1 with a sphere-shaped ending element 10. The sphere-shaped ending element 10 may have a diameter that is larger than the maximum clear width S formed by the redirection surface 6. The clear width S extends perpendicular to the longitudinal extend L at the second longitudinal end 4 and between opposite parts of the redirection surface 6.

The clear width S may correspond to the outer diameter E of the core 2. Alternatively, the clear width S may be smaller than the outer diameter E of the core 2. In case the clear width S is smaller than the outer diameter E of the core 2, the core 2 may comprise an end face 8 that extends perpendicular to the longitudinal extend L at the second longitudinal end 4. The end face 8 may contact the redirection surface 6. The end face 8 may even contact the and at least partly or even completely extends around the redirection surface 6 perpendicular to the longitudinal extend L. A groove or notch between the sphere-shaped ending element 10 and the end face 8 may be filled with a filler material, which may be an adhesive affixing the sphere-shaped ending element 10 to the end face 8.

The sphere-shaped ending element 10 may have a diameter such that it contacts the redirection surface 6 close to or at a line at which the redirection surface 6 contacts the end face 8. The lateral side 5 of the core 2 may be free from overlap from the ending element 10.

Figure 5 exemplarily shows the waveguide 1 with an ending element 10 that is formed as a lens. The core 2 may correspond to the core 2 of the exemplary embodiment of figure 4. The lens may have optical properties that direct light not redirected by the redirection surface 6 in a desired manner and may in particular disperse the light and may, hence, be a diverging, negative or diffuser lens.

The ending element 10 of figure 5 may have a convex or concave side that faces away from the core 2 in the longitudinal extend L. A side of the ending element 10 that faces the core 2 may be flat and/or may contact the end face 8 at least partly. For example, an edge of the lens may contact the end face 8. The end face 8 may be completely contacted by the side. The ending element 10 may be mounted and for example affixed and e.g. glued to the end face 8. The lateral side 5 of the core 2 may be free from overlap from the ending element 10.

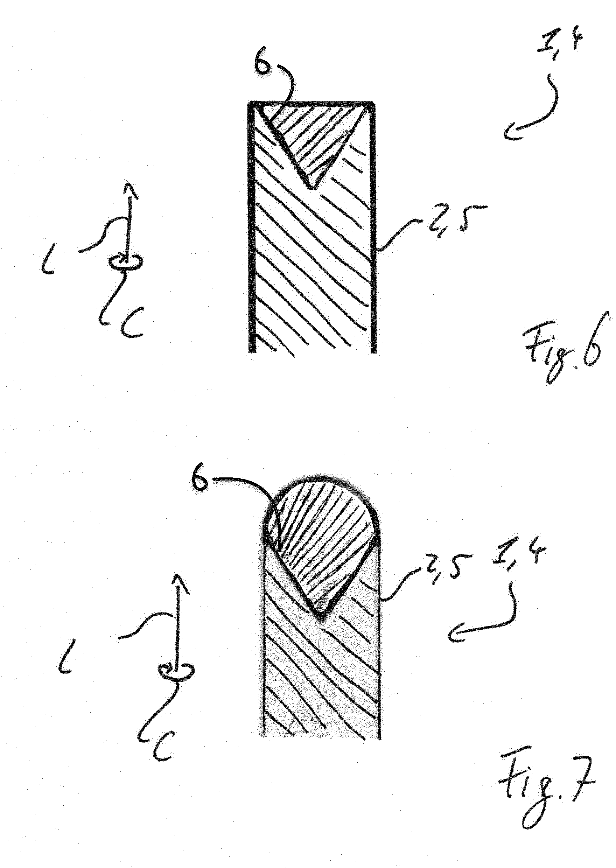

Figure 6 exemplarily shows the waveguide 1 with an ending element 10 that is formed as a coating on the redirection surface 6. The coating may be a reflective coating like a dielectric layer stack or a metal layer, the metal, for example, comprising or being a Nobel metal like gold.

Figure 7 exemplarily shows the waveguide 1 with an ending element 10 that is formed by casting. The ending element 10 can be cast into the recess formed by the redirection surface 6 and may at least partly or even completely fill the recess. Alternatively, the ending element 10 can be cast over the second longitudinal end 4 with the redirection surface 6 jutting the lateral side 5. Optionally or additionally, the cast ending element 10 may at least partly be formed with a convex or concave surface that faces away from the core 2 in the longitudinal extend L, the convex or concave surface being a lens surface. A surface of the cast ending element 10 that faces towards the core 2, i.e. against the longitudinal extend L, may at least partly or even completely be formed complementary to the redirection surface 6.

Alternatively, the ending element 10 may be formed with a plate-shape, wherein the ending element 10 may be mounted to the end face 8 in the same manner as the lens shaped ending element 10 of figure 5.

The material of the cast ending element 10 may have an optical property that differs from the respective optical property of the material of the core 2. For example, the optical property may be the refractive index. Particularly, the material of the cast ending element 10 may have a refractive index that is lower than the refractive index of the core material. For example, the material of the cast ending element 10 may be a so-called low-index material.

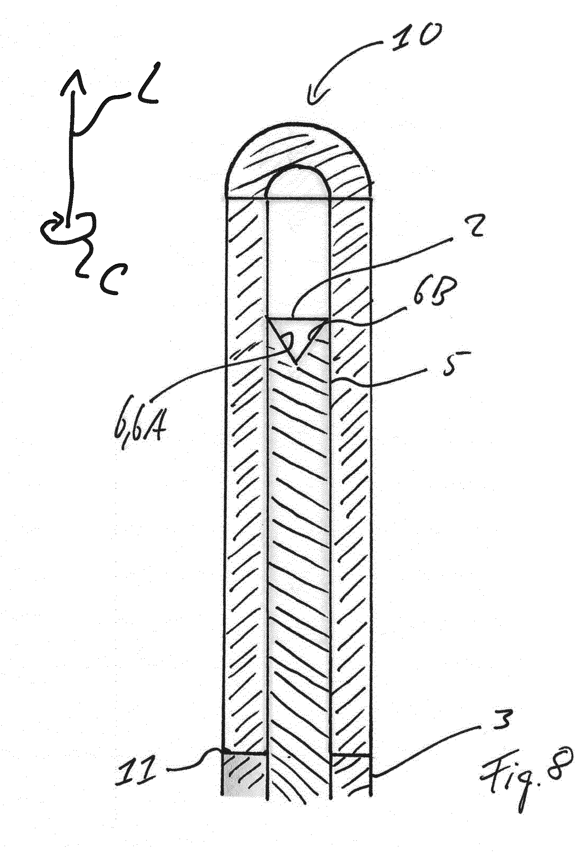

Figure 8 depicts another exemplary embodiment of the waveguide 1 in a schematic cross-sectional view. For elements, which in form and/or structure correspond to the elements of the exemplary embodiments of the previous figures, the same reference numerals are used. For the sake of brevity, only the differences with respect to the exemplary embodiments of the previous figures are mentioned below.

In particular, figure 8 shows the core 2 of the exemplary embodiment of figure 3 with the ending element 10 of figure 2. At least the ending elements 10 of the exemplary embodiments of any of figures 5, 6 or 7 may be added as additional ending element 10 to the exemplary embodiment of figure 8.

### Reference Signs

- 1: waveguide
- 2: core
- 3: coating
- 4: second longitudinal end
- 5: lateral side
- 6: redirection surface
- 6A,6B: redirection surface sections
- 7: end face of coating
- 8: end face of core
- 10: ending element
- 11: filler material
- 12: free volume
- 13: cladding

- C: circumferential direction
- d,D: distance
- E: outer diameter of the core
- h: length of free volume
- H: length of ending element
- L: longitudinal extend
- S: clear width of redirection surface
- W: angle

## Claims

1. Waveguide (1) for treating inner walls of inner pathways of a human body with energy waves, wherein the waveguide (1) comprises
a first longitudinal end for receiving energy waves,
a second longitudinal end (4) for redirecting the energy waves that travelled from the first longitudinal end through the waveguide (1) to the second longitudinal end (4),
the first and second (4) longitudinal ends being opposite ends of the waveguide (1) along a longitudinal extend (L) of the waveguide (1), and
a lateral side (5) that extends from the first longitudinal end to the second longitudinal end (4) along the longitudinal extend (L), wherein
the second longitudinal end (2) comprises at least one redirection surface (6) provided by the waveguide (2), the redirection surface (6) being unpolished,
**characterized in that**
the redirection surface (6) comprises a predefined roughness with a roughness parameter Rₐ between 0,5 µm and 10 µm.

2. Waveguide (1) according to claim 1, **characterized in that** the redirection surface (6) is formed by grinding the second longitudinal end (4) of the waveguide (4) and/or by laser treatment of the material of the second longitudinal end (4) of the waveguide (1) at least on the surface.

3. Waveguide (1) according to claim 1 or 2, **characterized in that** the redirection surface (6) juts the lateral side (5) in the longitudinal extend (L).

4. Waveguide (1) according to claim 1 or 2, **characterized in that** the redirection surface (6) forms a recess that extends along the longitudinal extend (L) from the second longitudinal end (4) towards the first longitudinal end.

5. Waveguide (1) according to any of claims 1 to 4, **characterized in that** the redirection surface (6) at least partly outlines a cone shape, a pyramid shape or a wedge shape.

6. Waveguide (1) according to any of claims 1 to 5, **characterized in that** the second longitudinal end (4) comprises an end face (7, 8) of the core (2) that is essentially aligned perpendicular to the longitudinal extend (L) at the second longitudinal end (4), wherein the end face (8) contacts the redirection surface (6), or
wherein the end face (7, 8) at least partly or even completely extends around the redirection surface (6) perpendicular to the longitudinal extend (L) of the waveguide (2) at the second longitudinal end (4), or
wherein the end face (8) contacts the redirection surface (6) and at least partly or even completely extends around the redirection surface (6) perpendicular to the longitudinal extend (L) of the waveguide (1) at the second longitudinal end (4), or
wherein the end face (7) is arranged at a distance to the redirection surface (6).

7. Waveguide (1) according to any of claims 1 to 6, **characterized in that** an ending element (10) is provided at the second longitudinal end (4).

8. Waveguide (1) according to claim 7 when depending from claim 6, **characterized in that** the ending element (10) overlaps the lateral side (5) and/or is mounted to the end face (7, 8).

9. Waveguide (1) according to claim 7 when depending from claim 6, **characterized in that** the lateral side (5) is free from overlap from the ending element (10) and/or the ending element (10) is mounted to the end face (7, 8).

10. Waveguide (1) according to any of claims 7 to 9, **characterized in that** a maximum diameter of the ending element (10) and a diameter of the waveguide (1) are essentially identical.

11. Waveguide (1) according to any of claims 7 to 10, **characterized in that** the ending element (10) comprises or is an ending cap, a sphere, a lens, a plate, an element formed by casting and/or a coating.

## Patentansprüche

1. Wellenleiter (1) zum Behandeln von Innenwänden von Innenbahnen eines menschlichen Körpers mit Energiewellen, wobei der Wellenleiter (1) Folgendes umfasst:
ein erstes Längsende zum Empfangen von Energiewellen,
ein zweites Längsende (4) zum Umlenken der Energiewellen, die vom ersten Längsende durch den Wellenleiter (1) zum zweiten Längsende (4) gelaufen sind,
wobei das erste und zweite (4) Längsende gegenüberliegende Enden des Wellenleiters (1) entlang einer Längserstreckung (L) des Wellenleiters (1) sind, und
eine Querseite (5), die sich entlang der Längserstreckung (L) vom ersten Längsende zum zweiten Längsende (4) erstreckt, wobei
das zweite Längsende (2) mindestens eine Umlenkfläche (6) umfasst, die der Wellenleiter (2) bereitstellt, wobei die Umlenkfläche (6) nicht poliert ist,
**dadurch gekennzeichnet, dass**
die Umlenkfläche (6) eine vordefinierte Rauheit mit einem Rauwert Rₐ zwischen 0,5 µm und 10 µm umfasst.

2. Wellenleiter (1) nach Anspruch 1, **dadurch gekennzeichnet, dass** die Umlenkfläche (6) durch Schleifen des zweiten Längsendes (4) des Wellenleiters (4) und/oder durch Laserbehandlung des Materials des zweiten Längsendes (4) des Wellenleiters (1) zumindest auf der Oberfläche ausgebildet ist.

3. Wellenleiter (1) nach Anspruch 1 oder 2, **dadurch gekennzeichnet, dass** die Umlenkfläche (6) in der Längserstreckung (L) aus der Querseite (5) ragt.

4. Wellenleiter (1) nach Anspruch 1 oder 2, **dadurch gekennzeichnet, dass** die Umlenkfläche (6) eine Aussparung ausbildet, die sich entlang der Längserstreckung (L) vom zweiten Längsende (4) in Richtung auf das erste Längsende erstreckt.

5. Wellenleiter (1) nach einem der Ansprüche 1 bis 4, **dadurch gekennzeichnet, dass** die Umlenkfläche (6) zumindest teilweise eine Kegelform, eine Pyramidenform oder eine Keilform begrenzt.

6. Wellenleiter (1) nach einem der Ansprüche 1 bis 5, **dadurch gekennzeichnet, dass** das zweite Längsende (4) eine Stirnfläche (7, 8) des Kerns (2) umfasst, die im Wesentlichen senkrecht zur Längserstreckung (L) am zweiten Längsende (4) ausgerichtet ist,
wobei die Stirnfläche (8) die Umlenkfläche (6) berührt, oder
wobei sich die Stirnfläche (7, 8) zumindest teilweise oder sogar vollständig um die Umlenkfläche (6) herum senkrecht zur Längserstreckung (L) des Wellenleiters (2) am zweiten Längsende (4) erstreckt, oder
wobei die Stirnfläche (8) die Umlenkfläche (6) berührt und sich zumindest teilweise oder sogar vollständig um die Umlenkfläche (6) herum senkrecht zur Längserstreckung (L) des Wellenleiters (1) am zweiten Längsende (4) erstreckt, oder
wobei die Stirnfläche (7) in einem Abstand zur Umlenkfläche (6) angeordnet ist.

7. Wellenleiter (1) nach einem der Ansprüche 1 bis 6, **dadurch gekennzeichnet, dass** am zweiten Längsende (4) ein Abschlusselement (10) vorgesehen ist.

8. Wellenleiter (1) nach Anspruch 7 bei Abhängigkeit von Anspruch 6, **dadurch gekennzeichnet, dass** das Abschlusselement (10) Querseite (5) überlappt und/oder an der Stirnfläche (7, 8) montiert ist.

9. Wellenleiter (1) nach Anspruch 7 bei Abhängigkeit von Anspruch 6, **dadurch gekennzeichnet, dass** die Querseite überlappungsfrei vom Abschlusselement (10) ist und/oder das Abschlusselement (10) an der Stirnfläche (7, 8) montiert ist.

10. Wellenleiter (1) nach einem der Ansprüche 7 bis 9, **dadurch gekennzeichnet, dass** ein Maximaldurchmesser des Abschlusselements (10) und ein Durchmesser des Wellenleiters (1) im Wesentlichen gleich sind.

11. Wellenleiter (1) nach einem der Ansprüche 7 bis 10, **dadurch gekennzeichnet, dass** das Abschlusselement (10) eine Endkappe, eine Kugel, eine Linse, eine Platte, ein durch Gießen geformtes Element und/oder eine Beschichtung umfasst oder ist.

## Revendications

1. Guide d'ondes (1) pour traiter des parois intérieures de voies intérieures d'un corps humain avec des ondes d'énergie, le guide d'ondes (1) comprenant
une première extrémité longitudinale pour recevoir des ondes d'énergie,
une deuxième extrémité longitudinale (4) pour rediriger les ondes d'énergie qui ont voyagé de la première extrémité longitudinale à travers le guide d'ondes (1) vers la deuxième extrémité longitudinale (4),
la première et la deuxième extrémité longitudinale (4) étant des extrémités opposées du guide d'ondes (1) le long d'une extension longitudinale (L) du guide d'ondes (1), et
un côté latéral (5) qui s'étend de la première extrémité longitudinale à la deuxième extrémité longitudinale (4) le long de l'extension longitudinale (L),
la deuxième extrémité longitudinale (2) comprenant au moins une surface de redirection (6) fournie par le guide d'ondes (2), la surface de redirection (6) n'étant pas polie,
**caractérisé en ce que**
la surface de redirection (6) comprend une rugosité prédéfinie avec un paramètre de rugosité Rₐ compris entre 0,5 µm et 10 µm.

2. Guide d'ondes (1) selon la revendication 1, **caractérisé en ce que** la surface de redirection (6) est formée par meulage de la deuxième extrémité longitudinale (4) du guide d'ondes (4) et/ou par traitement au laser du matériau de la deuxième extrémité longitudinale (4) du guide d'ondes (1) au moins sur la surface.

3. Guide d'ondes (1) selon la revendication 1 ou 2, **caractérisé en ce que** la surface de redirection (6) fait saillie sur le côté latéral (5) dans l'extension longitudinale (L).

4. Guide d'ondes (1) selon la revendication 1 ou 2, **caractérisé en ce que** la surface de redirection (6) forme un renfoncement qui s'étend le long de l'extension longitudinale (L) de la deuxième extrémité longitudinale (4) vers la première extrémité longitudinale.

5. Guide d'ondes (1) selon l'une quelconque des revendications 1 à 4, **caractérisé en ce que** la surface de redirection (6) dessine au moins partiellement une forme de cône, une forme de pyramide ou une forme de coin.

6. Guide d'ondes (1) selon l'une quelconque des revendications 1 à 5, **caractérisé en ce que** la deuxième extrémité longitudinale (4) comprend une face d'extrémité (7, 8) du noyau (2) qui est essentiellement alignée perpendiculairement à l'extension longitudinale (L) à la deuxième extrémité longitudinale (4),
la face d'extrémité (8) étant en contact avec la surface de redirection (6), ou
la face d'extrémité (7, 8) s'étendant au moins partiellement ou même complètement autour de la surface de redirection (6) perpendiculairement à l'extension longitudinale (L) du guide d'ondes (2) à la deuxième extrémité longitudinale (4), ou
la face d'extrémité (8) étant en contact avec la surface de redirection (6) et s'étendant au moins partiellement ou même complètement autour de la surface de redirection (6) perpendiculairement à l'extension longitudinale (L) du guide d'ondes (1) à la deuxième extrémité longitudinale (4), ou
la face d'extrémité (7) étant disposée à une certaine distance de la surface de redirection (6).

7. Guide d'ondes (1) selon l'une quelconque des revendications 1 à 6, **caractérisé en ce qu'**un élément de terminaison (10) est prévu à la deuxième extrémité longitudinale (4).

8. Guide d'ondes (1) selon la revendication 7 lorsqu'elle dépend de la revendication 6, **caractérisé en ce que** l'élément de terminaison (10) recouvre le côté latéral (5) et/ou est monté sur la face d'extrémité (7, 8).

9. Guide d'ondes (1) selon la revendication 7 lorsqu'elle dépend de la revendication 6, **caractérisé en ce que** le côté latéral (5) est exempt de chevauchement avec l'élément de terminaison (10) et/ou l'élément de terminaison (10) est monté sur la face d'extrémité (7, 8).

10. Guide d'ondes (1) selon l'une quelconque des revendications 7 à 9, **caractérisé en ce qu'**un diamètre maximal de l'élément de terminaison (10) et un diamètre du guide d'ondes (1) sont essentiellement identiques.

11. Guide d'ondes (1) selon l'une quelconque des revendications 7 à 10, **caractérisé en ce que** l'élément de terminaison (10) comprend ou est un capuchon de terminaison, une sphère, une lentille, une plaque, un élément formé par moulage et/ou un revêtement.
